# EUROPÄISCHE PATENTSCHRIFT

(11) **EP 0 987 229 B1**
(45) Veröffentlichungstag und Bekanntmachung des Hinweises auf die Patenterteilung: **27.03.2002**
(21) Anmeldenummer: 99117629.8
(22) Anmeldetag: 07.09.1999
(51) Int. Cl.: C03C 29/00, A61N 1/05

(54) **Biokompatible Glas-Metall-Durchführungen und ihre Verwendung**
Biocompatible glass-metal passages and their use
Traversées verre-métal biocompatibles et leur application

(30) Priorität: 18.09.1998 DE 19842943
(43) Veröffentlichungstag der Anmeldung: 22.03.2000
(73) Patentinhaber: Schott Glas, 55122 Mainz (DE); Carl-Zeiss-Stiftung trading as Schott Glas, 55122 Mainz (DE)
(72) Erfinder: Stehlik, Vojtech, 84030 Landshut (DE); Puscher, Oswald, 85408 Gammelsdorf (DE)

(56) Entgegenhaltungen:
- EP-A- 0 137 488
- GB-A- 952 660
- US-A- 3 770 568
- US-A- 4 220 813
- US-A- 5 633 531

## Beschreibung

Die Erfindung betrifft biokompatible Glas-Metall-Durchführungen und ihre Verwendung in implantierbaren medizinischen Geräten und Vorrichtungen sowie die Geräte und Vorrichtungen selbst.

Glas-Metall-Durchführungen sind in den verschiedensten Anwendungsbereichen von großer Bedeutung, so z.B. in der Elektrotechnik. Hier ist insbesondere die Langzeithermetizität der Durchführungen elektrischer Leiter in Gehäusen für Bauelemente der Elektronik, speziell auch der Opto- und der Kfz-Elektronik, und der Sensorik gefordert, die Schmelzverbindungen der Gläser mit verschiedenen Metallen notwendig macht.

Eine Besonderheit bei Glas-Metall-Durchführungen in hermetisch abgedichteten Komponenten stellen implantierbare, biokompatible Durchführungen dar. Sie werden eingesetzt bei implantierbaren medizinischen Vorrichtungen, z.B. Herzschrittmachern. Wesentlich für diese Durchführungen sind die Bioverträglichkeit, eine hohe Korrosionsfestigkeit sowie eine gute Langzeitbeständigkeit aller eingesetzten Komponenten.

Bekannt sind implantierbare, biokompatible Durchführungen, die aus einem Außenleiter aus Titan, einem biokompatiblen Glas und einem Innenleiter aus Tantal bestehen.

Glas ist aufgrund seiner Langzeitbeständigkeit, seiner guten Verarbeitbarkeit und aufgrund der Möglichkeit, seinen Wärmeausdehnungskoeffizienten über die Zusammensetzung in einem gewissen Bereich zu variieren und so eine angepaßte oder eine sogenannte Druck-Glas-Durchführung mit dichter Schmelzverbindung zum jeweiligen Metall zu ermöglichen, als Werkstoff für hermetische Durchführungen sehr gut geeignet. An ein zu implantierendes Glas werden natürlich besondere Anforderungen hinsichtlich seiner Bioverträglichkeit, also letztlich hinsichtlich seiner Zusammensetzung gestellt. Gläser, die keine toxischen Bestandteile haben, oder nur so geringe Mengen, daß sie physiologisch unbedenklich sind, sind z.B. in der Patentschrift DE 40 15 264 C1 beschrieben. Sie werden auch als Kapselmaterial für implantierbare Minisender oder Transponder verwendet.

Tantal ist aufgrund seiner chemischen Resistenz und seiner Biokompatibilität als geeigneter Werkstoff für Innenleiter bekannt.

Für viele implantierbare medizinische Vorrichtungen, so auch als Außenleiter bei den genannten Glas-Metall-Durchführungen, wird Titan verwendet. Dieses Metall besitzt eine sehr gute Korrosionsfestigkeit und eine hohe Biokompatibilität.

Die derzeit produzierten biokompatiblen Glas-Metall-Durchführungen sind jedoch besonders aufgrund der Verwendung von Titan in ihrer Produktion sehr aufwendig. Mit dem Ziel einer umfassenden medizinischen Versorgung, zu der heutzutage infolge des hohen Kenntnisstands in Medizin und Medizintechnik auch immer selbstverständlicher Implantationen von beispielsweise Herzschrittmachern gehören, ist eine kostengünstige Zurverfügungstellung der Implantate bzw. ihrer Bestandteile erstrebenswert.

Es ist Aufgabe der Erfindung, biokompatible, implantierbare, hermetische Glas-Metall-Durchführungen zu finden, die, insbesondere aufgrund ihrer Produktionsprozesse und der verwendeten Werkstoffe, kostengünstig produzierbar sind.

Diese Aufgabe wird durch die im Patentanspruch 1 beschriebenen Glas-Metall-Durchführungen gelöst.

Sie sind aufgebaut aus einem Außenleiter, einem biokompatiblen Glas und wenigstens einem Innenleiter.

Der Außenleiter besteht aus einem nickelfreien rostfreien, chemisch beständigen Stahl (Edelstahl). Es wurde nämlich überraschenderweise gefunden, daß solche Stähle sowohl ausreichend korrosionsfest und biokompatibel sind, als auch aufgrund ihrer weiteren Werkstoffeigenschaften wie gute Zerspanbarkeit und gute Schweißbarkeit geeignet sind, die bisher verwendeten, teuren Titanleiter zu ersetzen. Unter nickelfrei ist hier ein Gehalt von max. 0,3 % Ni zu verstehen, was auch die medizinisch indizierten Anforderungen an Nikkelfreiheit erfüllt. Im Rahmen der medizinischen Zulassungsverfahren werden die weiteren medizinisch indizierten Anforderungen in umfangreichen Tests überprüft werden. Die jeweiligen Anforderungen können aufgrund fortschreitenden Wissens und verbesserter Analysemethoden Wandlungen unterworfen sein. Solche Edelstähle sind an sich bekannt. Beispielhaft seien hier ein austenitischer Edelstahl (a) und ein ferritischer Edelstahl (b) genannt:
a) Rostfreier Stahl, bestehend aus 0 - 0,1 % C; 16,0 - 20,0 % Cr; 16,0 - 20,0 % Mn; 1,8 - 2,5 % Mo; 0,7 - 1,0 % N; 0 - 0,3 % Ni, 0 - 0,05 % P; 0 - 0,05 % S; 0 - 1,0 % Si; 0 - 0,2 % V; Rest einschließlich erschmelzungsbedingter Verunreinigungen Eisen.
b) Rostfreier Stahl, bestehend aus 0 - 0,03 % C; 17,5 - 18,5 % Cr; 0 - 0,5 % Mn; 2,0 - 2,5 % Mo; 0 - 0,3 % Ni; 0 - 0,03 % P; 0,15 - 0,35 % S; 0 - 1,0 % Si; 0,3 - 1,0 % Ti; Rest einschließlich erschmelzungsbedingter Verunreinigungen Eisen.

Die Verwendung solcher gängigen, preisgünstigen Edelstähle in den erfindungsgemäßen Durchführungen verbilligt deren Produktion enorm.

Als Dichtmittel und elektrischer Isolator zum Einglasen können beliebige biokompatible Gläser verwendet werden. Solche Gläser sind gewebeverträglich, verursachen keine Abstoßreaktionen im Körper und sind ausreichend korrosionsfest. '
Als Beispiele seien die Gläser folgender Zusammensetzungsbereiche (in Gew.-% auf Oxidbasis) genannt:
- 40 - 45 SiO₂; 1 - 4 B₂O₃; 35 - 40 Al₂O₃; 0 - 2 BaO; 4 - 10 CaO; 4 - 10 MgO; 4 - 10 P₂O₅; 0 - 1 MnO₂
- 65 - 70 SiO₂; 0 - 1 B₂O₃; 3 - 5 Al₂O₃; 11 - 15 Na₂O; 2 - 4 K₂O; 0 - 2 BaO; 4 - 6 CaO; 2 - 5 Fe₂O₃; 2 - 4 MgO
- 64-70 SiO₂; 15 - 22 B₂O₃; 0 - 3 Al₂O₃; 0 - 1 Li₂O; 0 - 1 Na₂O; 5 - 11 K₂O; 0 - 1 ZnO.

Als Innenleiter kann Tantal verwendet werden. Auch die Verwendung von anderen für die Einschmelzung geeigneten Werkstoffen mit einem thermischen Ausdehnungskoeffizienten α_{200/400} zwischen 40 x 10⁻⁷ und 110 x 10⁻⁷ K⁻¹ ist möglich. Hier sind z. B. nickelfreie rostfreie ferritische Edelstähle zu nennen, beispielsweise AISI 446 (US-Norm) oder ähnliche ferritische Edelstähle. Innenleiter aus Edelstahl sind bisher nur im allgemeinen Industriebereich, beispielsweise bei Kühlschranksockeln bekannt. Gut geeignet aufgrund ihrer Biokompatibilität und ihrer guten Lötbarkeit sind auch Platin und Platinlegierungen. Hier sind insbesondere Pt/Ir-Legierungen zu nennen. Bei diesen Legierungen beträgt der Iridium-Anteil meist 5 - 30 %.

Die erfindungsgemäßen elektrischen Durchführungen sind hervorragend geeignet für die Verwendung in Implantaten wie z.B. Herzschrittmacher und andere Vorrichtungen für funktionelle Elektrostimulation, beispielsweise Gehörimplantate, Gehirnschrittmacher, Defibrilatoren und in der dynamischen Myoplastie, Atemschrittmacher, Bein- und Handschrittmacher. Ihre Verwendung ist aber nicht auf diese Anwendungsgebiet beschränkt; sie können genausogut beispielsweise in der industriellen Sensorik oder auf ähnlichen Gebieten eingesetzt werden.

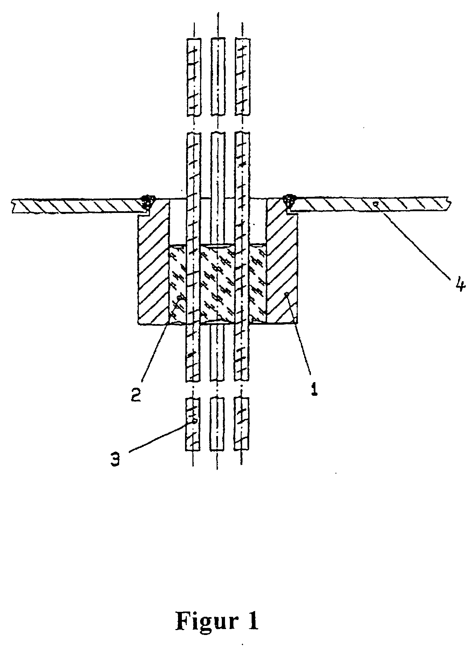

Der Aufbau der erfindungsgemäßen Glas-Metall-Durchführungen wird im folgenden anhand der Zeichnung näher erläutert. Figur 1 zeigt beispielhaft in schematischer Darstellung einen Schnitt durch eine Ausführungsform der Erfindung. Die erfindungsgemäße Materialkombination ist selbstverständlich nicht auf diese Ausführungsform beschränkt, sondern geeignet für sämtliche, dem Fachmann bekannte Formen von Glas-Metall-Durchführungen, so auch beispielsweise für solche, die einen multilayer-chip als Kondensator (EMV-Filter) enthalten.

Figur 1 zeigt eine Glas-Metall-Durchführung, deren Außenleiter 1 ringförmig ausgebildet ist und aus einem nickelfreien rostfreien, chemisch beständigen Stahl (Edelstahl) der oben genannten Zusammensetzung b) (ferritischer Edelstahl Sandvik 1802 von der Firma Sandvik Steel) besteht. In ihm befindet sich zentriert der vierpolige Innenleiter 3. Die Stromleiter-Drähte nehmen ausgezeichnete Positionen auf einer vierzähligen Drehachse ein. Auch ihre eigenen Achsen sind eingezeichnet. Die Drähte bestehen aus Tantal, können aber auch beispielsweise aus Platin oder Platin/lridium-Legierungen bestehen. Der Innenleiter 3 ist eingeschmolzen in einen isolierenden Glaskörper 2, der bis zu einer Höhe von etwa 2/3 den Raum innerhalb des Außenleiters 1 ausfüllt (selbstverständlich wäre auch ein vollständiges Einglasen möglich) und mit diesem ebenfalls verschmolzen ist. Bei dem Glas handelt es sich um ein biokompatibles Glas aus dem oben als zweites genannten Zusammensetzungsbereich. An den Außenleiter 1 ist bereits das Metallgehäuse 4 des Geräts, in dem die Durchführung Verwendung findet, angeschweißt.

Die erfindungsgemäßen biokompatiblen Glas-Metall-Durchführungen entsprechen bzgl. aller Anforderungen, die an solche Durchführungen gestellt werden, den bisher für Implantate verwendeten Durchführungen und zeichnen sich dadurch aus, daß sie aufgrund der als Außenleiter-Material verwendeten Edelstähle kostengünstiger produzierbar sind als die bisher bekannten Durchführungen.

## Patentansprüche

1. Biokompatible Glas-Metall-Durchführung,
bestehend aus einem Außenleiter, einem biokompatiblen Glas und wenigstens einem Innenleiter,
**dadurch gekennzeichnet,**
**daß** der Außenleiter aus einem nickelfreien, das heißt mit einem Ni-Gehalt zwischen 0 und 0,3 %, rostfreien, chemisch beständigen Stahl (Edelstahl) besteht.

2. Glas-Metall-Durchführung nach Anspruch 1,
**dadurch gekennzeichnet,**
**daß** der/die Innenleiter aus Tantal besteht/bestehen.

3. Glas-Metall-Durchführung nach Anspruch 1,
**dadurch gekennzeichnet,**
**daß** der/die Innenleiter aus Platin oder Platinlegierungen besteht/bestehen.

4. Glas-Metall-Durchführung nach Anspruch 1,
**dadurch gekennzeichnet,**
**daß** der/die Innenleiter aus einem nickelfreien rostfreien ferritischen Edelstahl besteht/bestehen.

5. Verwendung der Glas-Metall-Durchführung nach wenigstens einem der Ansprüche 1 bis 4 in implantierbaren medizinischen Geräten oder Vorrichtungen.

6. Implantierbare(s) medizinische(s) Vorrichtung oder Gerät mit wenigstens einer Glas-Metall-Durchführung nach wenigstens einem der Ansprüche 1 bis 4.

## Claims

1. Biocompatible glass-metal leadthrough, combining an outer conductor, a biocompatible glass and at least one inner conductor, **characterized in that** the outer conductor consists of a nickel-free (i.e. having a Ni content of between 0 and 0.3%), stainless, chemically resistant steel (special steel).

2. Glass-metal leadthrough according to Claim 1, **characterized in that** the inner conductor(s) consist(s) of tantalum.

3. Glass-metal leadthrough according to Claim 1, **characterized in that** the internal conductor(s) consist(s) of platinum or platinum alloys.

4. Glass-metal leadthrough according to Claim 1, **characterized in that** the internal conductor(s) consist(s) of a nickel-free, stainless ferritic special steel.

5. Use of the glass-metal leadthrough according to at least one of Claims 1 to 4 in implantable medical appliances or devices.

6. Implantable medical device or appliance having at least one glass-metal leadthrough according to at least one of Claims 1 to 4.

## Revendications

1. Traversée verre-métal biocompatible, se composant d'un conducteur extérieur, d'un verre biocompatible et d'au moins un conducteur intérieur, **caractérisée en ce que** le conducteur extérieur est constitué d'un acier sans nickel, c'est-à-dire avec une teneur en nickel comprise entre 0 et 0,3 %, inoxydable et résistant aux substances chimiques (acier allié).

2. Traversée verre-métal suivant la revendication 1, **caractérisée en ce que** le/les conducteur(s) intérieur(s) est/sont constitué(s) de tantale.

3. Traversée verre-métal suivant la revendication 1, **caractérisée en ce que** le/les conducteur(s) intérieur(s) est/sont constitué(s) de platine ou d'alliages de platine.

4. Traversée verre-métal suivant la revendication 1, **caractérisée en ce que** le/les conducteur(s) intérieur(s) est/sont constitué(s) d'un acier allié ferritique inoxydable sans nickel.

5. Utilisation d'une traversée verre-métal suivant au moins une des revendications 1 à 4 dans des appareils ou des dispositifs médicaux à implanter.

6. Dispositif ou appareil médical à implanter, avec au moins une traversée verre-métal suivant au moins une des revendications 1 à 4.
